(19) Europäisches Patentamt
European Patent Office
Office européen des brevets

(11) EP 4 253 954 A1

(12) EUROPEAN PATENT APPLICATION
published in accordance with Art. 153(4) EPC

(43) Date of publication:
**04.10.2023 Bulletin 2023/40**

(21) Application number: **21915120.6**

(22) Date of filing: **17.12.2021**

(51) International Patent Classification (IPC):
***G01N 33/50*** *(2006.01)*

(52) Cooperative Patent Classification (CPC):
**G01N 33/50**

(86) International application number:
**PCT/JP2021/046814**

(87) International publication number:
**WO 2022/145260 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.12.2020 JP 2020219130**

(71) Applicant: **Hamamatsu Photonics K.K.**
**Hamamatsu-shi, Shizuoka 435-8558 (JP)**

(72) Inventors:
- **OHBA, Hiroyuki**
  **Hamamatsu-shi, Shizuoka 435-8558 (JP)**
- **TSUKADA, Hideo**
  **Hamamatsu-shi, Shizuoka 435-8558 (JP)**

(74) Representative: **Grünecker Patent- und Rechtsanwälte PartG mbB**
**Leopoldstraße 4**
**80802 München (DE)**

# (54) METHOD FOR ASSESSING MITOCHONDRIAL FUNCTION IN TISSUE OR ORGAN OTHER THAN KIDNEY IN TEST SUBJECT

(57) The present invention relates to a method for assessing mitochondrial function in a tissue or organ other than the kidney in a test subject, including: a step of calculating a ratio (BUN/CRE) of blood urea nitrogen (BUN) concentration to blood creatinine (CRE) concentration in a test subject; and a step of estimating mitochondrial activity in a tissue or organ other than the kidney in the test subject using the calculated ratio (BUN/CRE).

*Fig.7*

(A)
UPTAKES IN HEART (SUV)
R2=0.121 P=0.027
BUN/CRE
(B)
R2=0.015 P=0.382
BLOOD GLUCOSE (mg/dL)
(C)
R2=0.063 P=0.068
BLOOD TOTAL CHOLESTEROL (mg/dL)
(D)
R2=0.065 P=0.065
BLOOD TRIGLYCERIDE (mg/dL)
(E)
R2=0.068 P=0.059
BLOOD INSULIN (ng/dL)



Processed by Luminess, 75001 PARIS (FR)

## Description

### Technical Field

The present invention relates to a method for assessing mitochondrial function in a tissue or organ other than the kidney in a test subject. The present invention also relates to a data collection method for estimating mitochondrial activity in a tissue or organ other than the kidney in a test subject.

### Background Art

Mitochondria are present in almost all cells that make up living organisms, and are sometimes called "the power plants of cells." Mitochondria play an important role in producing adenosine triphosphate (ATP), which is an essential energy source for cell activity, from sugars and lipids obtained from digestion of ingested food. For this reason, it is not hard to imagine that the state of health of a living body, which is an aggregate of cells, depends greatly on the active state of mitochondria.
Positron emission tomography (PET) probes for assessing mitochondrial function are known (for example, Non Patent Literature 1).

### Citation List

### Patent Literature

[Non Patent Literature 1] J. Nucl. Med., 2020, vol. 61, pp. 96-103
[Non Patent Literature 2] Life Sciences, 2005, vol. 76, pp. 1825-1834
[Non Patent Literature 3] Clin. Kidney J., 2012, vol. 5, pp. 187-191
[Non Patent Literature 4] Intensive Care Med., 2019, vol. 45, pp. 1813-1815
[Non Patent Literature 5] Nephrology Reviews, 2010, vol. 2, e11

### Summary of Invention

### Technical Problem

The PET probe (for example, $[^{18}F]$BCPP-EF) disclosed in Non Patent Literature 1 can specifically measure mitochondrial complex I (MC-I). By performing PET measurement using such a PET probe, noninvasive measurement becomes possible, and the burden on the test subject can be reduced. On the other hand, PET measurement requires large-scale facilities and specialists in each field for the production of positron-emitting nuclides by a cyclotron, label synthesis of PET probes, collection of image data, quantitative analysis of images, and the like. For this reason, the method for assessing mitochondrial function by PET measurement is by no means highly versatile, and there is a demand for an assessment method that can be implemented more simply.
Accordingly, an object of the present invention is to provide a method for assessing mitochondrial function more simply.

### Solution to Problem

The present inventors have found that the ratio (BLTN/CRE) calculated from the blood urea nitrogen (BUN) concentration and blood creatinine (CRE) concentration, which are used as indices for assessing kidney function, surprisingly shows a good correlation with mitochondrial activity in a tissue or organ other than the kidney. The present invention is based on this new finding.
The present invention relates to a method for assessing the mitochondrial function in a tissue or organ other than the kidney in a test subject, including: a step of calculating a ratio (BUN/CRE) of blood urea nitrogen (BUN) concentration to blood creatinine (CRE) concentration in a test subject; and a step of estimating mitochondrial activity in a tissue or organ other than the kidney in the test subject using the calculated ratio (BUN/CRE).
It has been proposed that the BUN/CRE value can more accurately assess kidney function than each of the conventionally used values of the blood urea nitrogen (BUN) concentration or blood creatinine (CRE) concentration (for example, Non Patent Literature 2 to 5). However, as described in the examples below, the BUN/CRE value is not universally recognized as an assessment index of kidney function, while there is a significant negative correlation with

the mitochondrial activity in a tissue or organ other than the kidney. Since the method according to the present invention uses the BUN/CRE value as an index, it is possible to assess the mitochondrial function in a tissue or organ other than the kidney. In addition, blood urea nitrogen (BUN) concentration and blood creatinine (CRE) concentration can be measured using blood collected from a test subject as a specimen, for example, using a generally used automated biochemical analyzer, and thus this method can be easily implemented.

The present invention also relates to a data collection method for estimating mitochondrial activity in a tissue or organ other than the kidney in a test subject, including: a step of calculating a ratio (BUN/CRE) of blood urea nitrogen (BUN) concentration to blood creatinine (CRE) concentration in a test subject.

In the above method, the tissue or organ may be at least one selected from the group consisting of brain, brown adipose tissue, heart, pancreas, and liver.

**Advantageous Effects of Invention**

According to the present invention, the method for assessing mitochondrial function more simply can be provided.

**Brief Description of Drawings**

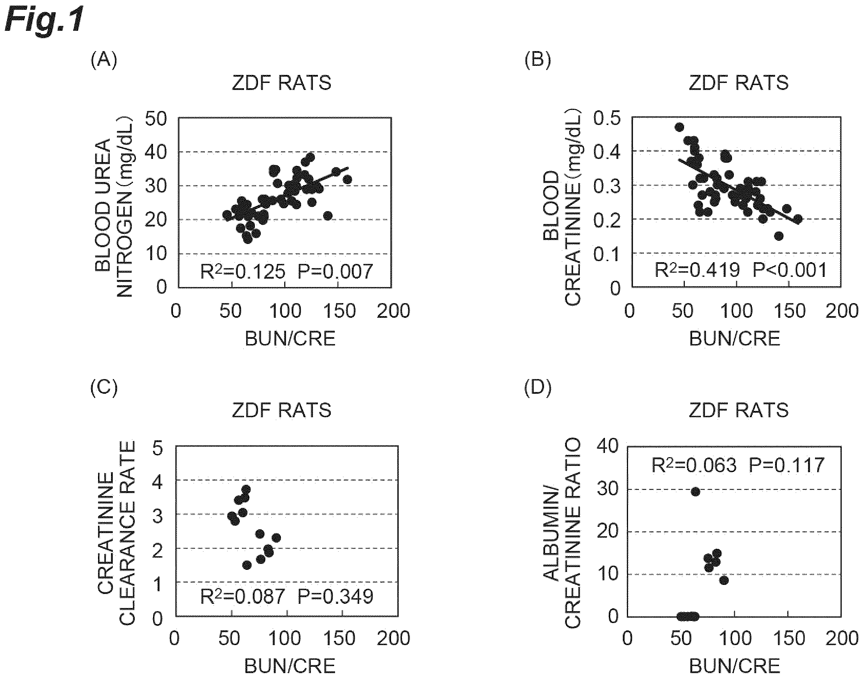

FIGS. 1(A), 1(B), 1(C), and 1(D) are graphs plotting each biochemical index value (FIG. 1(A): blood urea nitrogen concentration, FIG. 1(B): blood creatinine concentration, FIG. 1(C): creatinine clearance rate (CCr), and FIG. 1(D): albumin/creatinine ratio) with respect to a BUN/CRE value. Each plot corresponds to measured and calculated values for ZDF rats and control rats.

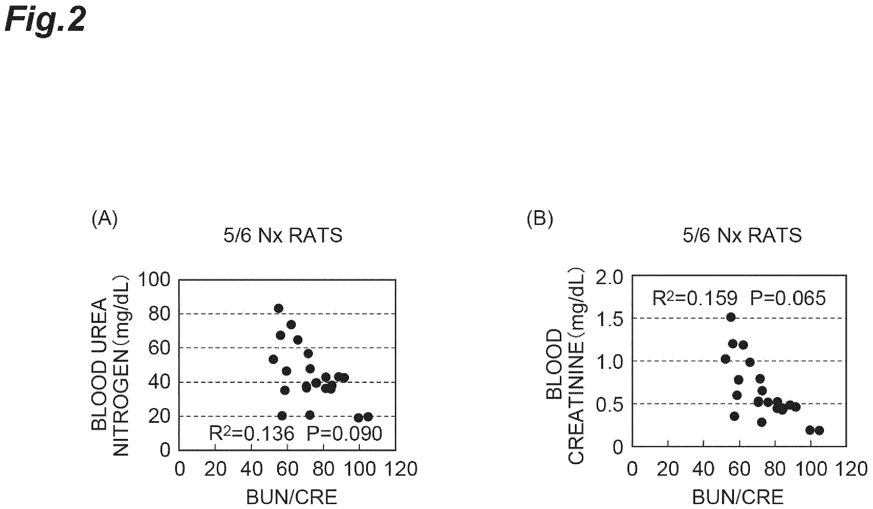

FIGS. 2(A) and 2(B) are graphs plotting each biochemical index value (FIG. 2(A): blood urea nitrogen concentration and FIG. 2(B): blood creatinine concentration) with respect to the BUN/CRE values. Each plot corresponds to measured and calculated values for 5/6Nx rats and control rats.

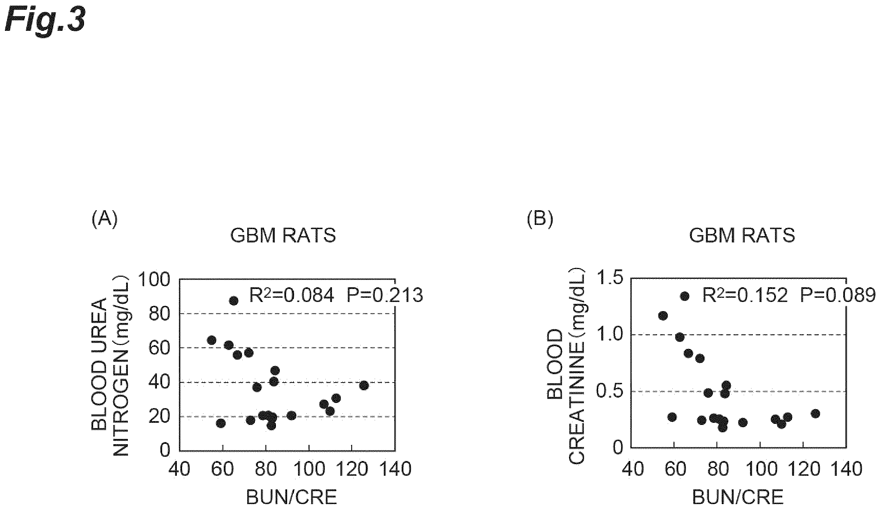

FIGS. 3(A) and 3(B) are graphs plotting each biochemical index value (FIG. 3(A): blood urea nitrogen concentration and FIG. 3(B): blood creatinine concentration) with respect to the BUN/CRE values. Each plot corresponds to measured and calculated values for GBM rats and control rats.

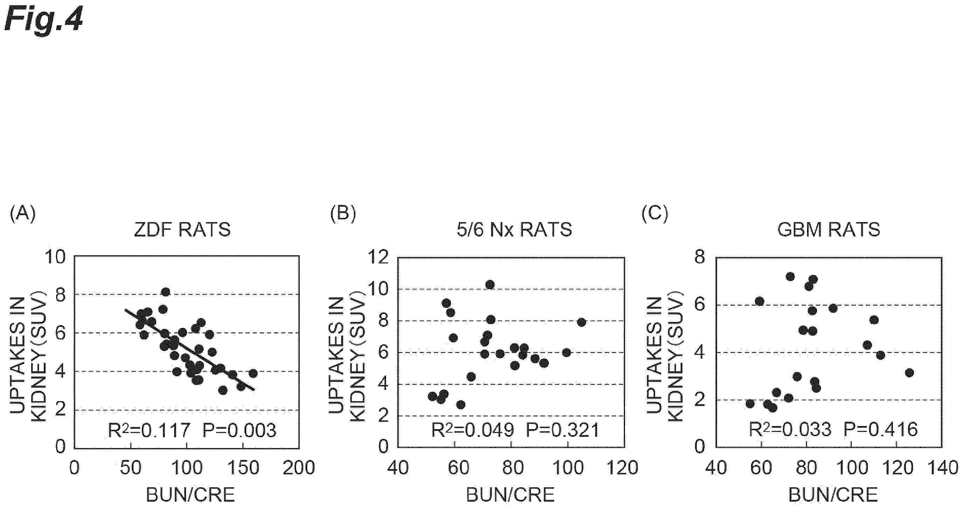

FIGS. 4(A), 4(B), and 4(C) are graphs plotting [$^{18}$F]BCPP-BF uptakes (SUV) in the kidney with respect to the BUN/CRE values. Each plot of FIG. 4(A) corresponds to measured and calculated values for ZDF rats and control rats. Each plot of FIG. 4(B) corresponds to measured and calculated values for 5/6Nx rats and control rats. Each plot of FIG. 4(C) corresponds to measured and calculated values for GBM rats and control rats.

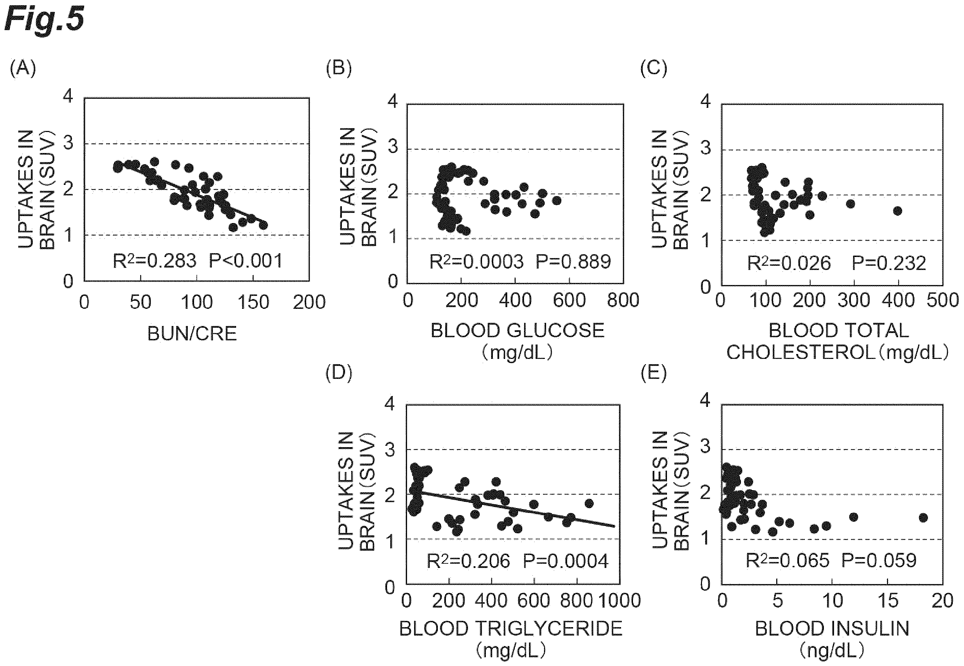

FIGS. 5(A), 5(B), 5(C), 5(D), and 5(E) are graphs plotting [$^{18}$F]BCPP-BF uptakes (SUV) in the brain with respect to each biochemical index value (FIG. 5(A): BUN/CRE, FIG. 5(B): blood glucose concentration, FIG. 5(C): blood total cholesterol concentration, FIG. 5(D): blood triglyceride concentration, and FIG. 5(E): blood insulin concentration). Each plot corresponds to measured and calculated values for ZDF rats and control rats.

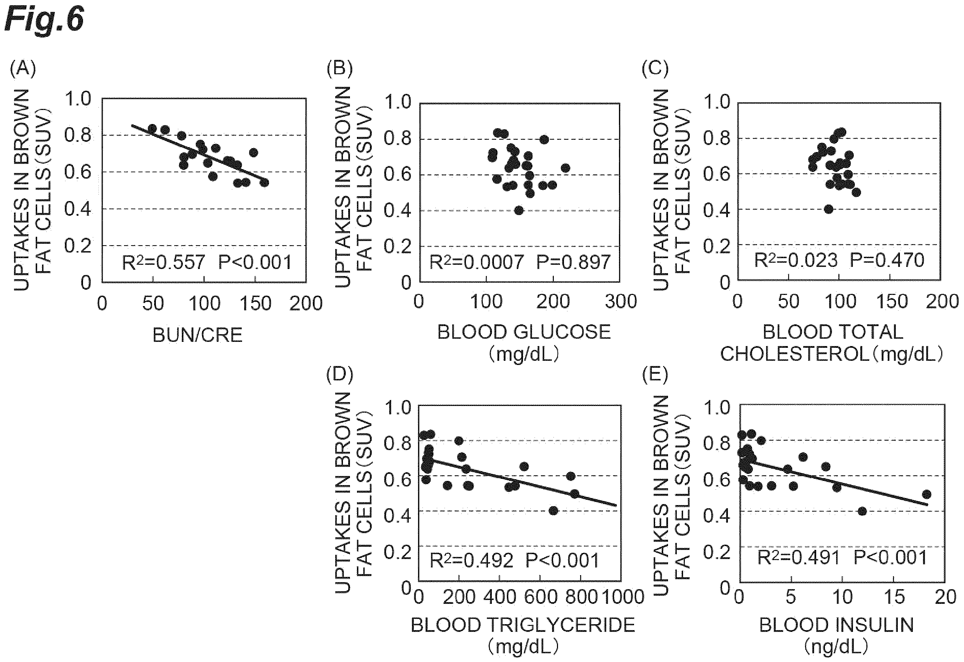

FIGS. 6(A), 6(B), 6(C), 6(D), and 6(E) are graphs plotting [$^{18}$F]BCPP-BF uptakes (SUV) in the brown fat cells (tissues) with respect to each biochemical index value (FIG. 6(A): BUN/CRE, FIG. 6(B): blood glucose concentration, FIG. 6(C): blood total cholesterol concentration, FIG. 6(D): blood triglyceride concentration, and FIG. 6(E): blood insulin concentration). Each plot corresponds to measured and calculated values for ZDF rats and control rats.

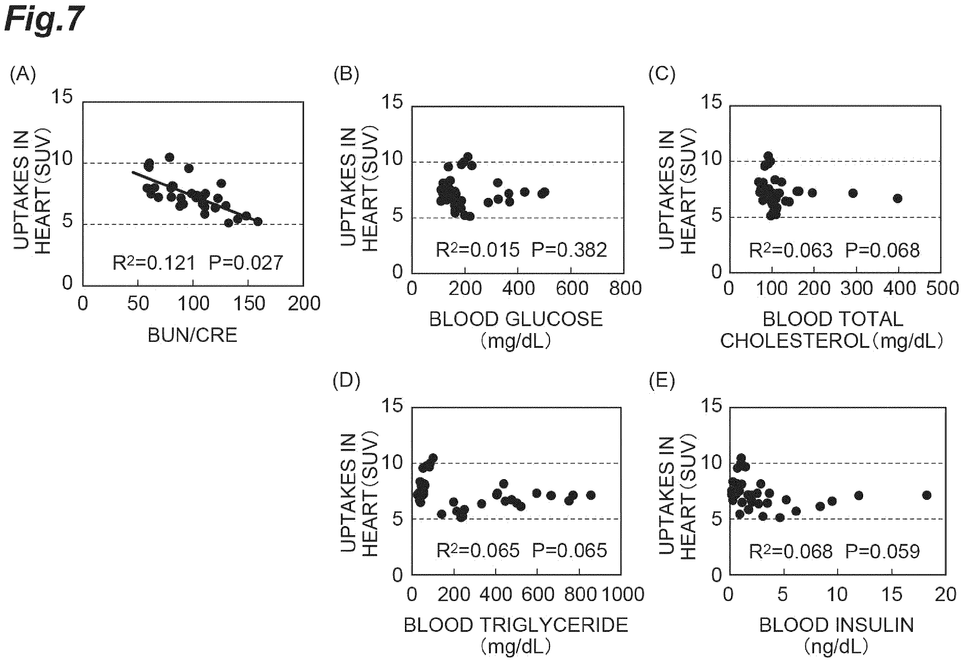

FIGS. 7(A), 7(B), 7(C), 7(D), and 7(E) are graphs plotting [$^{18}$F]BCPP-BF uptakes (SUV) in the heart with respect to each biochemical index value (FIG. 7(A): BUN/CRE, FIG. 7(B): blood glucose concentration, FIG. 7(C): blood total cholesterol concentration, FIG. 7(D): blood triglyceride concentration, and FIG. 7(E): blood insulin concentration). Each plot corresponds to measured and calculated values for ZDF rats and control rats.

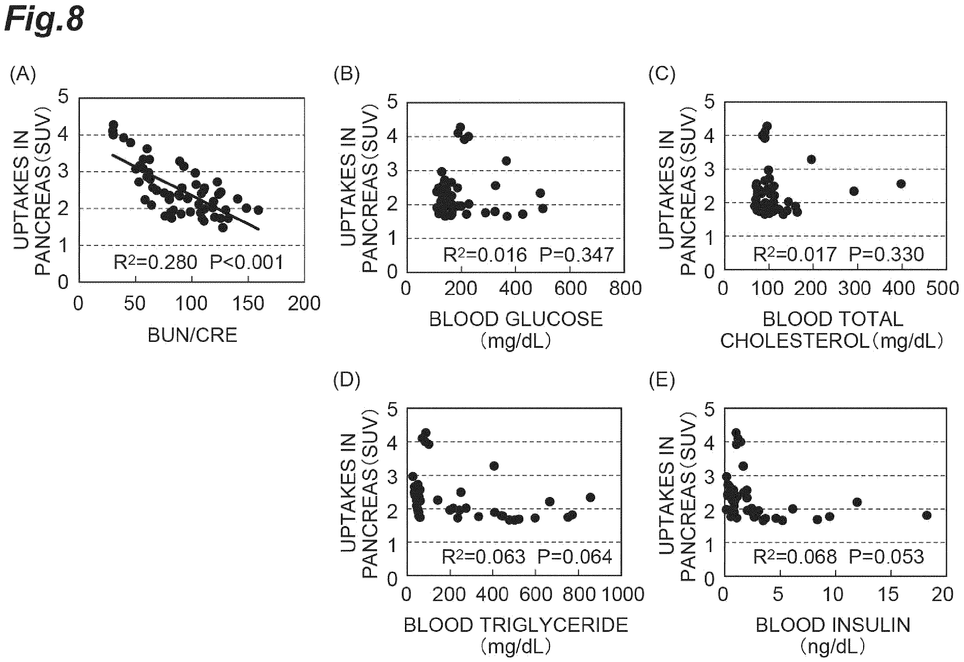

FIGS. 8(A), 8(B), 8(C), 8(D), and 8(E) are graphs plotting [$^{18}$F]BCPP-BF uptakes (SUV) in the liver with respect to each biochemical index value (FIG. 8(A): BUN/CRE, FIG. 8(B): blood glucose concentration, FIG. 8(C): blood total cholesterol concentration, FIG. 8(D): blood triglyceride concentration, and FIG. 8(E): blood insulin concentration). Each plot corresponds to measured and calculated values for ZDF rats and control rats.

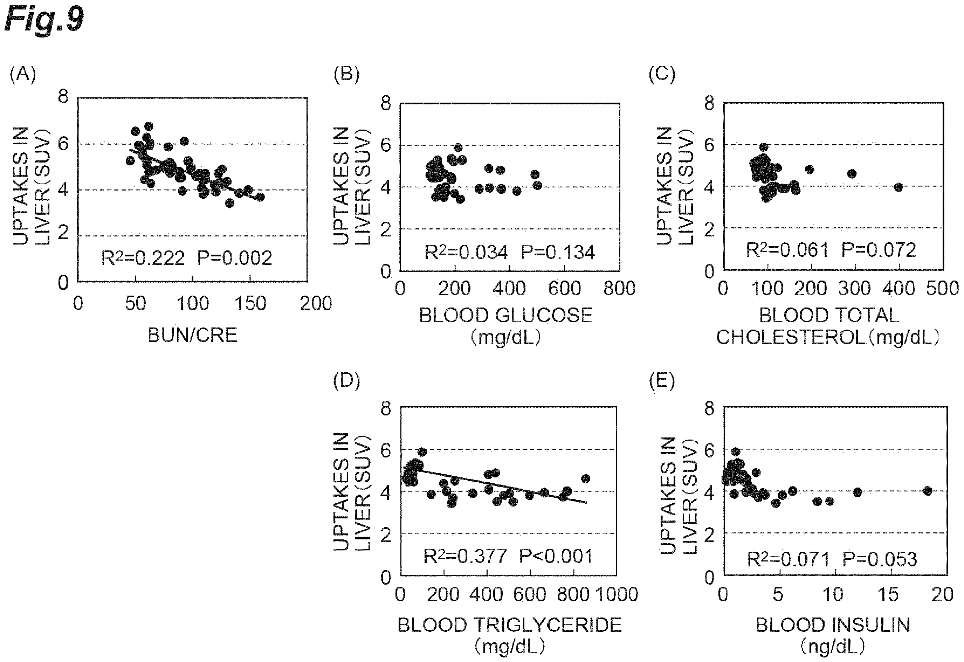

FIGS. 9(A), 9(B), 9(C), 9(D), and 9(E) are graphs plotting [$^{18}$F]BCPP-BF uptakes (SUV) in the liver with respect to each biochemical index value (FIG. 9(A): BUN/CRE, FIG. 9(B): blood glucose concentration, FIG. 9(C): blood total cholesterol concentration, FIG. 9(D): blood triglyceride concentration, and FIG. 9(E): blood insulin concentration). Each plot corresponds to measured and calculated values for ZDF rats and control rats.

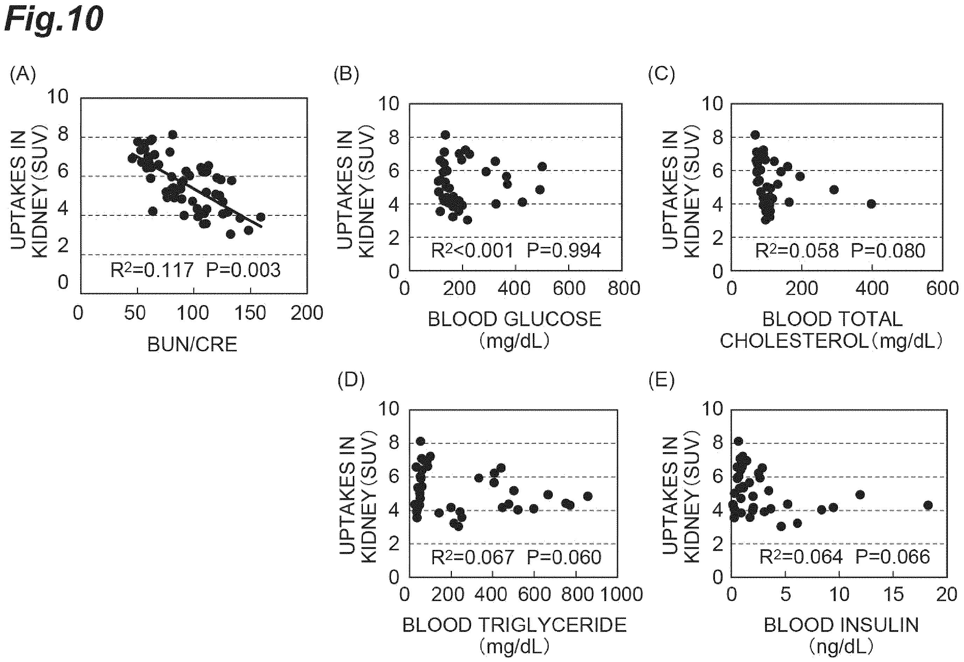

FIGS. 10(A), 10(B), 10(C), 10(D), and 10(E) are graphs plotting [$^{18}$F]BCPP-BF uptakes (SUV) in the kidney with respect to each biochemical index value (FIG. 10(A): BUN/CRE, FIG. 10(B): blood glucose concentration, FIG. 10(C): blood total cholesterol concentration, FIG. 10(D): blood triglyceride concentration, and FIG. 10(E): blood insulin concentration). Each plot corresponds to measured and calculated values for ZDF rats and control rats.

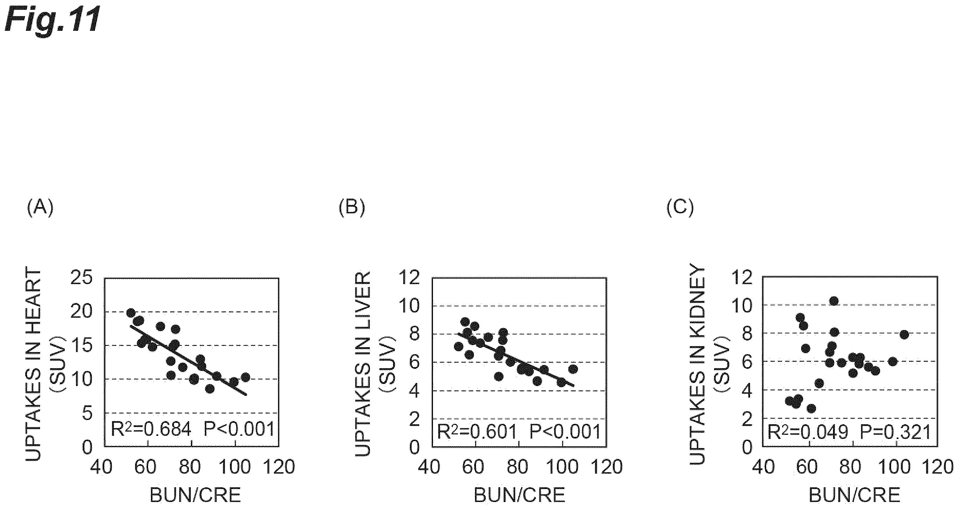

FIGS. 11(A), 11(B), and 11(C) are graphs plotting [$^{18}$F]BCPP-BF uptakes (SUV) in each organ (FIG. 11(A): heart, FIG. 11(B): liver, and FIG. 11(C): kidney) with respect to BUN/CRE values. Each plot corresponds to measured and calculated values for 5/6Nx rats and control rats.

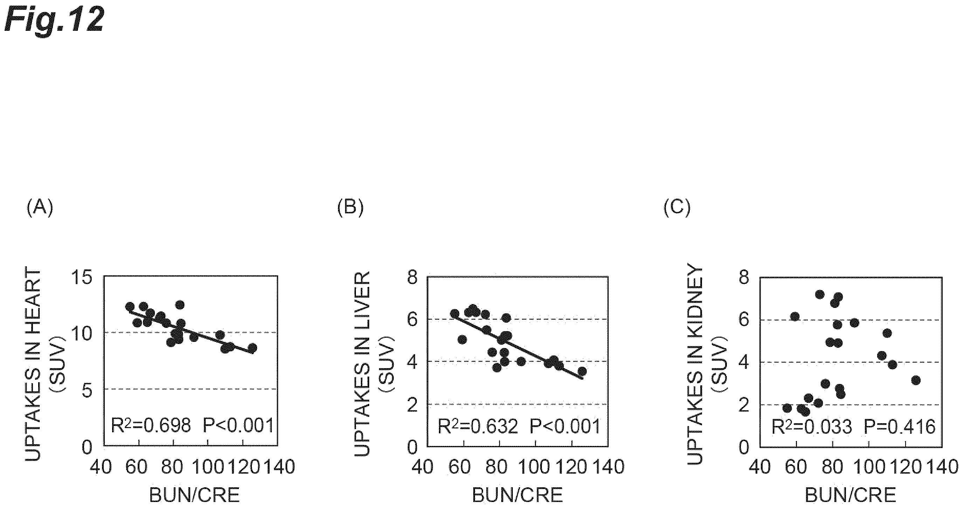

FIGS. 12(A), 12(B), and 12(C) are graphs plotting [$^{18}$F]BCPP-BF uptakes (SUV) in each organ (FIG. 12(A): heart, FIG. 12(B): liver, and FIG. 12(C): kidney) with respect to BUN/CRE values. Each plot corresponds to measured and calculated values for GBM rats and control rats.

## Description of Embodiments

Hereinafter, embodiments for carrying out the present invention will be described in detail. However, the present invention is not limited to the following embodiments.

[Method for assessing mitochondrial function]

A method for assessing mitochondrial function in a tissue or organ other than the kidney in a test subject (hereinafter also simply referred to as "assessment method") according to the present embodiment, includes: a step of calculating a ratio (BUN/CRE) of blood urea nitrogen (BUN) concentration to blood creatinine (CRE) concentration in a test subject (calculation step); and a step of estimating mitochondrial activity in a tissue or organ other than the kidney in the test subject using the calculated ratio (BUN/CRE) (estimation step).

The assessment method according to the present embodiment may further include a step of measuring blood urea nitrogen (BUN) concentration using blood collected from a test subject as a specimen (BUN measurement step); and/or a step of measuring blood creatinine (CRE) concentration using blood collected from the test subject as a specimen (CRE measurement step).

Blood urea nitrogen (BUN) concentration can be measured according to a conventional method. Specifically, for example, measurement can be performed by detecting nitrogen contained in urea using blood itself collected from a test subject, or serum or plasma separated from blood. Nitrogen contained in urea can be detected using, for example, a commercially available kit. Further, detection of nitrogen contained in urea may be performed using an automated biochemical analyzer or the like.

Blood creatinine (CRE) concentration can be measured according to a conventional method. Specifically, for example, measurement can be performed by an enzymatic method (creatinase-sarcosine oxidase-peroxidase method) using blood itself collected from a test subject, or serum or plasma separated from blood. The blood creatinine concentration can be measured using, for example, a commercially available kit. In addition, blood creatinine concentration may be measured using an automated biochemical analyzer or the like.

In the calculation step, the ratio (BUN/CRE) of blood urea nitrogen (BUN) concentration to blood creatinine (CRE) concentration in the test subject is calculated. Specifically, for example, BUN/CRE can be calculated by dividing the blood urea nitrogen concentration (mg/dL) measured in the BUN measurement step by the blood creatinine concentration (mg/dL) measured in the CRE measurement step.

In the estimation step, the calculated ratio (BUN/CRE) is used to estimate mitochondrial activity in a tissue or organ other than the kidney in the test subject. Since the BUN/CRE value shows a negative correlation with mitochondrial activity in a tissue or organ other than the kidney, in the estimation step, using this correlation, the mitochondrial activity in each of tissues or organs (excluding the kidney) of the test subject can be estimated from the calculated ratio (BUN/CRE).

Mitochondrial activity in each of tissues or organs (excluding the kidney) of a test subject can be estimated by, for example, deriving a relationship between BUN/CRE values and the mitochondrial activity value in each of tissues or organs (excluding the kidney) from a plurality of data pairs (BUN/CRE values and mitochondrial activity values in each of tissues or organs (excluding the kidney)) obtained in advance and using the derived relationship and the BUN/CRE values obtained for the test subject. More specifically, mitochondrial activity in each of tissues or organs (excluding the kidney) of the test subject can be estimated, for example, by calculating a correlation formula from a plurality of data pairs obtained in advance and introducing the BUN/CRE value measured in the test subject into the correlation formula. In addition, the correlation may be derived by machine learning using a huge amount of data.

The estimated value of mitochondrial activity obtained in the estimation step can also be used to determine whether or not the test subject has a disorder or disease in a particular organ or tissue (excluding the kidney). In this case, for example, the relationship between the BUN/CRE value and the mitochondrial activity value in the tissue or organ is derived from one or more data pairs obtained in advance from a healthy test subject and one or more data pairs obtained in advance from a test subject having a disorder or disease in a specific tissue or organ (excluding the kidney), and a threshold value that can distinguish between healthy test subjects and test subjects having disorders or diseases in the relevant tissue or organ is obtained in advance. Then, by comparing the estimated value of mitochondrial activity obtained in the estimation step with the threshold value, it is possible to determine whether or not the test subject has

a disorder or disease in a specific tissue or organ (excluding the kidney). In addition, it may be determined whether or not a person has a disorder or a disease based on the result of machine learning from a huge amount of data.
Test subjects may be, for example, humans, monkeys, mice, and rats, but are preferably humans.
Tissue or organ, which are assessment targets, are not particularly limited as long as they are tissues or organs other than the kidney. Specifically, examples thereof include brain, brown fat cell (brown adipose tissue), heart, pancreas, liver, and muscle. The tissue or organ, which are assessment targets, may be, for example, at least one selected from the group consisting of brain, brown fat cell (brown adipose tissue), heart, pancreas, and liver.

[Data collection method]

A data collection method according to the present embodiment is a method for collecting data for estimating mitochondrial activity in a tissue or organ other than the kidney in a test subject, at least including: a step of calculating a ratio (BUN/CRE) of blood urea nitrogen (BUN) concentration to blood creatinine (CRE) concentration in the test subject. As described above, the BUN/CRE value obtained by the data collection method according to the present embodiment can be used to estimate the mitochondrial activity in a tissue or organ other than the kidney in the test subject.
The data collection method according to the present embodiment may further include a step of measuring blood urea nitrogen (BUN) concentration using blood collected from a test subject as a specimen (BUN measurement step); and/or a step of measuring blood creatinine (CRE) concentration using blood collected from the test subject as a specimen (CRE measurement step).

**Example**

The present invention will be described in more detail below based on examples. However, the present invention is not limited to these.

[Test Example 1: Assessment of mitochondrial complex I activity]

(Synthesis of PET probe)

[$^{18}$F]BCPP-BF represented by the following formula was synthesized according to the method described in Non Patent Literature (J. Labeled Comp. Radiopharm., 2013, vol. 56, no. 11, pp. 553-561). The final product obtained had a radiochemical purity of 99.0% and a specific radioactivity of 73.4 GBq/μmol.

[Chemical 1]

It is known that [$^{18}$F]BCPP-BF can be used to detect mitochondrial complex I (MC-I). [$^{18}$F]BCPP-BF accumulates specifically in MC-I, and the uptake which is proportional to the MC-I activity in the target tissue or organ is obtained, and thus [$^{18}$F]BCPP-BF uptake can be used as an index to assess MC-I activity in the target tissue or organ.
In order to identify the location of the pancreas, a probe (D-[$^{11}$C]MT) that recognizes the amino acid transporter (LAT-1) highly expressed in the pancreas of small animals was prepared. D-[$^{11}$C]MT was synthesized by the method described in Example 1 of WO2005/115971. The final product obtained had a radiochemical purity of 100.0% and a specific radioactivity of 56.4 GBq/μmol.

(ZDF rat)

Male Zucker Lepr$^{fa}$/Lepr$^{fa}$ rats (hereinafter also referred to as "ZDF rat") that develop a pathology similar to adult human type II diabetes were purchased from Charles River Laboratories Japan, Inc., and PET measurement was

performed at 5 weeks old, 8 weeks old, 16 weeks old, and 26 weeks old. As controls, male Zucker Lepr$^{fa}$/+ rats (hereinafter also referred to as "control rats") were purchased from Charles River Laboratories Japan, Inc. and used.

(5/6Nx rat)

As a chronic kidney disease model, rats with 5/6 of kidney removed (excision treatment at 7 weeks of age, hereinafter also referred to as "5/6Nx rat") were purchased from Japan SLC Inc., and test subjected to PET measurement at 2 weeks and 14 weeks after the excision treatment. As controls, sham-operated rats without nephrectomy were used.

(GBM rat)

As an acute kidney disease model, rats with nephritis administered glomerular basement membrane antibody (anti-GBM antibody) (administration treatment at 7 weeks of age, hereinafter also referred to as "GBM rat") were purchased from Japan SLC Inc., and test subjected to PET measurement at 2 weeks and 6 weeks after the administration treatment. As controls, sham-operated rats without administration of anti-GBM antibody were used.

(PET measurement)

Rats were anesthetized with isoflurane and fixed in the gantry of an animal PET camera (SHR-38000, manufactured by Hamamatsu Photonics K.K.). After performing a 15-minute transmission measurement for absorption correction, approximately 20 MBq/0.5 mL of D-[$^{11}$C]MT was administered to the rats via the tail vein, and a 60-minute emission measurement was performed. Subsequently, approximately 20 MBq/0.5 mL of [$^{18}$F]BCPP-BF was administered through the tail vein of the rat, and emissions were measured for 60 minutes.

After the PET measurement was completed, a region of interest was set on the pancreas identified from the PET uptake images 40 to 60 minutes after administration of D-[$^{11}$C]MT, and the [$^{18}$F]BCPP-BF uptake in the region of interest was calculated. Then, the calculated uptake was normalized by the body weight of each individual and the amount of administered radioactivity, and was defined as the [$^{18}$F]BCPP-BF uptake in the pancreas (radioactivity uptake (SUV)). For tissue or organ other than the pancreas and the brain, a region of interest was set for each of tissues or organs identified in the PET uptake image of [$^{18}$F]BCPP-BF, and the radioactivity uptake (SUV) was calculated in the same manner as for the pancreas. The radioactivity uptake (SUV) of the brain was calculated by removing the brain from the rat immediately after the PET measurement.

[Test Example 2: Measurement of biochemical indices]

Blood was collected from the rat immediately after the PET measurement, and blood urea nitrogen concentration, blood creatinine concentration, blood glucose concentration, blood total cholesterol concentration, blood triglyceride concentration, and blood insulin concentration were measured using an automated biochemical analyzer (7180 manufactured by Hitachi High-Tech Corporation). Moreover, the BUN/CRE value was calculated by the following formula.

$$\text{BUN/CRE} = \text{blood urea nitrogen concentration (mg/dL)/blood creatinine concentration (mg/dL)}$$

The rat urine was collected immediately after the PET measurement, and the urine albumin concentration and urine creatinine concentration were measured using an automated biochemical analyzer (7180 manufactured by Hitachi High-Tech Corporation). In addition, the creatinine clearance rate (CCr) and the albumin/creatinine ratio (ACR) were calculated by the following formulas.

$$\text{CCr} = \text{(urine creatinine concentration (mg/dL)} \times \text{urine volume per hour (dL))/blood creatinine concentration (mg/dL)}$$

$$\text{ACR} = \text{urine albumin concentration (mg/dL)/urine creatinine concentration (mg/dL)}$$

[Test Example 3: Correlation analysis between MC-I activity and biochemical indices]

The correlation between the radioactivity uptake (SUV) data obtained in Test Example 1 and the biochemical index data obtained in Test Example 2 was analyzed.

FIGS. 1(A), 1(B), 1(C), and 1(D) are graphs plotting each biochemical index value (FIG. 1(A): blood urea nitrogen concentration, FIG. 1(B): blood creatinine concentration, FIG. 1(C): creatinine clearance rate (CCr), and FIG. 1(D): albumin/creatinine ratio) with respect to a BUN/CRE value. Each plot corresponds to measured and calculated values for ZDF rats and control rats.

FIGS. 2(A) and 2(B) are graphs plotting each biochemical index value (FIG. 2(A): blood urea nitrogen concentration and FIG. 2(B): blood creatinine concentration) with respect to the BUN/CRE values. Each plot corresponds to measured and calculated values for 5/6Nx rats and control rats.

FIGS. 3(A) and 3(B) are graphs plotting each biochemical index value (FIG. 3(A): blood urea nitrogen concentration and FIG. 3(B): blood creatinine concentration) with respect to the BUN/CRE values. Each plot corresponds to measured and calculated values for GBM rats and control rats.

In ZDF rats, the values of blood urea nitrogen concentration and blood creatinine concentration and the BUN/CRE values showed significant positive and negative correlations, respectively (FIGS. 1(A) and 1(B)), but showed no correlation with the values of creatinine clearance rate and albumin/creatinine ratio, which directly reflect kidney function (FIGS. 1(C) and 1(D)). In 5/6Nx rats, the values of blood urea nitrogen concentration and blood creatinine concentration and the BUN/CRE values showed no correlation (FIGS. 2(A) and 2(B)). Similarly, in GBM rats, the values of blood urea nitrogen concentration and blood creatinine concentration and the BUN/CRE values showed no correlation (FIGS. 3(A) and 3(B)). These results suggest that the BUN/CRE value is not universally used as an index of kidney function.

FIGS. 4(A), 4(B), and 4(C) are graphs plotting [$^{18}$F]BCPP-BF uptakes (SUV) in the kidney with respect to the BUN/CRE values. Each plot of FIG. 4(A) corresponds to measured and calculated values for ZDF rats and control rats. Each plot of FIG. 4(B) corresponds to measured and calculated values for 5/6Nx rats and control rats. Each plot of FIG. 4(C) corresponds to measured and calculated values for GBM rats and control rats.

MC-I activity in the kidney, which was assessed by [$^{18}$F]BCPP-BF uptake (SUV), showed a significant negative correlation with the BUN/CRE values in ZDF rats (FIG. 4(A)), but 5/6Nx and GBM rats showed no correlation (FIGS. 4(B) and 4(C)). These results also suggest that the BUN/CRE value is not universally used as an index of kidney function.

FIGS. 5(A), 5(B), 5(C), 5(D), and 5(E) are graphs plotting [$^{18}$F]BCPP-BF uptakes (SUV) in the brain with respect to values (FIG. 5(A): BUN/CRE, FIG. 5(B): blood glucose concentration, FIG. 5(C): blood total cholesterol concentration, FIG. 5(D): blood triglyceride concentration, and FIG. 5(E): blood insulin concentration) of each biochemical index. Each plot corresponds to measured and calculated values for ZDF rats and control rats.

MC-I activity in the brain of ZDF rats, which was assessed by [$^{18}$F]BCPP-BF uptake (SUV), showed a significant negative correlation only with the BUN/CRE values (FIG. 5(A)) and the blood triglyceride concentration values (FIG. 5(D)).

FIGS. 6(A), 6(B), 6(C), 6(D), and 6(E) are graphs plotting [$^{18}$F]BCPP-BF uptakes (SUV) in the brown fat cells (tissues) with respect to values (FIG. 6(A): BUN/CRE, FIG. 6(B): blood glucose concentration, FIG. 6(C): blood total cholesterol concentration, FIG. 6(D): blood triglyceride concentration, and FIG. 6(E): blood insulin concentration) of each biochemical index. Each plot corresponds to measured and calculated values for ZDF rats and control rats.

MC-I activity in the brown fat cells (tissues) of ZDF rats, which was assessed by [$^{18}$F]BCPP-BF uptake (SUV), showed a significant negative correlation only with the BUN/CRE values (FIG. 6(A)), the blood triglyceride concentration values (FIG. 6(D)), and the blood insulin concentration values (FIG. 6(E)).

FIGS. 7(A), 7(B), 7(C), 7(D), and 7(E) are graphs plotting [$^{18}$F]BCPP-BF uptakes (SUV) in the heart with respect to values (FIG. 7(A): BUN/CRE, FIG. 7(B): blood glucose concentration, FIG. 7(C): blood total cholesterol concentration, FIG. 7(D): blood triglyceride concentration, and FIG. 7(E): blood insulin concentration) of each biochemical index. Each plot corresponds to measured and calculated values for ZDF rats and control rats.

MC-I activity in the heart of ZDF rats, which was assessed by [$^{18}$F]BCPP-BF uptake (SUV), showed a significant negative correlation only with the BUN/CRE values (FIG. 7(A)).

FIGS. 8(A), 8(B), 8(C), 8(D), and 8(E) are graphs plotting [$^{18}$F]BCPP-BF uptakes (SUV) in the liver with respect to values (FIG. 8(A): BUN/CRE, FIG. 8(B): blood glucose concentration, FIG. 8(C): blood total cholesterol concentration, FIG. 8(D): blood triglyceride concentration, and FIG. 8(E): blood insulin concentration) of each biochemical index. Each plot corresponds to measured and calculated values for ZDF rats and control rats.

MC-I activity in the liver of ZDF rats, which was assessed by [$^{18}$F]BCPP-BF uptake (SUV), showed a significant negative correlation only with the BUN/CRE values (FIG. 8(A)).

FIGS. 9(A), 9(B), 9(C), 9(D), and 9(E) are graphs plotting [$^{18}$F]BCPP-BF uptakes (SUV) in the liver with respect to values (FIG. 9(A): BUN/CRE, FIG. 9(B): blood glucose concentration, FIG. 9(C): blood total cholesterol concentration, FIG. 9(D): blood triglyceride concentration, and FIG. 9(E): blood insulin concentration) of each biochemical index. Each plot corresponds to measured and calculated values for ZDF rats and control rats.

MC-I activity in the liver of ZDF rats, which was assessed by [$^{18}$F]BCPP-BF uptake (SUV), showed a significant

negative correlation only with the BUN/CRE values (FIG. 9(A)) and the blood triglyceride concentration values (FIG. 9(D)).

FIGS. 10(A), 10(B), 10(C), 10(D), and 10(E) are graphs plotting [$^{18}$F]BCPP-BF uptakes (SUV) in the kidney with respect to values (FIG. 10(A): BUN/CRE, FIG. 10(B): blood glucose concentration, FIG. 10(C): blood total cholesterol concentration, FIG. 10(D): blood triglyceride concentration, and FIG. 10(E): blood insulin concentration) of each biochemical index. Each plot corresponds to measured and calculated values for ZDF rats and control rats.

MC-I activity in the kidney of ZDF rats, which was assessed by [$^{18}$F]BCPP-BF uptake (SUV), showed a significant negative correlation only with the BUN/CRE values (FIG. 10(A)).

FIGS. 11(A), 11(B), and 11(C) are graphs plotting [$^{18}$F]BCPP-BF uptakes (SUV) in each organ (FIG. 11(A): heart, FIG. 11(B): liver, and FIG. 11(C): kidney) with respect to BUN/CRE values. Each plot corresponds to measured and calculated values for 5/6Nx rats and control rats.

The BUN/CRE values showed a significant negative correlation with MC-I activity in the heart and the liver of 5/6Nx rats, which was assessed by [$^{18}$F]BCPP-BF uptake (SUV) (FIGS. 11(A) and 11(B)), and showed no significant correlation with MC-I activity in the kidney (FIG. 11(C)).

FIGS. 12(A), 12(B), and 12(C) are graphs plotting [$^{18}$F]BCPP-BF uptakes (SUV) in each organ (FIG. 12(A): heart, FIG. 12(B): liver, and FIG. 12(C): kidney) with respect to BUN/CRE values. Each plot corresponds to measured and calculated values for GBM rats and control rats.

The BUN/CRE values showed a significant negative correlation with MC-I activity in the heart and the liver of GBM rats, which was assessed by [$^{18}$F]BCPP-BF uptake (SUV), (FIGS. 12(A) and 12(B)), and showed no significant correlation with MC-I activity in the kidney (FIG. 12(C)).

(Summary)

As shown in the test examples above, the BUN/CRE value was not universally recognized as an assessment index of kidney function. On the other hand, it was found that the BUN/CRE value showed a significant negative correlation with MC-I activity in a tissue or organ other than the kidney, which was assessed by [$^{18}$F]BCPP-BF uptake (SUV), and the BUN/CRE value can be used as an assessment index of mitochondrial function in each organ (tissue or organ other than the kidney).

## Claims

**1.** A method for assessing mitochondrial function in a tissue or organ other than the kidney in a test subject, comprising:

a step of calculating a ratio (BLTN/CRE) of blood urea nitrogen (BUN) concentration to blood creatinine (CRE) concentration in a test subject; and
a step of estimating mitochondrial activity in a tissue or organ other than the kidney in the test subject using the calculated ratio (BUN/CRE).

**2.** A data collection method for estimating mitochondrial activity in a tissue or organ other than the kidney in a test subject, comprising:
a step of calculating a ratio (BLTN/CRE) of blood urea nitrogen (BUN) concentration to blood creatinine (CRE) concentration in a test subject.

**3.** The method according to claim 1 or 2, wherein the tissue or organ is at least one selected from the group consisting of brain, brown adipose tissue, heart, pancreas, and liver.

*Fig.1*

(A)

ZDF RATS

BLOOD UREA NITROGEN (mg/dL)

0 10 20 30 40 50

$R^2$=0.125 P=0.007

0 50 100 150 200

BUN/CRE

(B)

ZDF RATS

BLOOD CREATININE (mg/dL)

0 0.1 0.2 0.3 0.4 0.5

$R^2$=0.419 P<0.001

0 50 100 150 200

BUN/CRE

(C)

ZDF RATS

CREATININE CLEARANCE RATE

0 1 2 3 4 5

$R^2$=0.087 P=0.349

0 50 100 150 200

BUN/CRE

(D)

ZDF RATS

ALBUMIN/ CREATININE RATIO

0 10 20 30 40

$R^2$=0.063 P=0.117

0 50 100 150 200

BUN/CRE

*Fig.2*

(A)
5/6 Nx RATS
BLOOD UREA NITROGEN (mg/dL)
100
80
60
40
20
0
R2=0.136 P=0.090
0 20 40 60 80 100 120
BUN/CRE
(B)
5/6 Nx RATS
BLOOD CREATININE (mg/dL)
2.0
1.5
1.0
0.5
0
R2=0.159 P=0.065
0 20 40 60 80 100 120
BUN/CRE

Fig.3

(A)
GBM RATS
R2=0.084 P=0.213
BLOOD UREA NITROGEN (mg/dL)
100
80
60
40
20
0
40
60
80
100
120
140
BUN/CRE
(B)
GBM RATS
R2=0.152 P=0.089
BLOOD CREATININE (mg/dL)
1.5
1.0
0.5
0
40
60
80
100
120
140
BUN/CRE

Fig.4

(A)
ZDF RATS
UPTAKES IN KIDNEY(SUV)
BUN/CRE
R2=0.117 P=0.003
0
2
4
6
8
10
0
50
100
150
200
(B)
5/6 Nx RATS
UPTAKES IN KIDNEY(SUV)
BUN/CRE
R2=0.049 P=0.321
0
2
4
6
8
10
12
40
60
80
100
120
(C)
GBM RATS
UPTAKES IN KIDNEY(SUV)
BUN/CRE
R2=0.033 P=0.416
0
2
4
6
8
40
60
80
100
120
140

Fig.5
(A)
UPTAKES IN BRAIN (SUV)
R2=0.283 P<0.001
BUN/CRE
(B)
UPTAKES IN BRAIN (SUV)
R2=0.0003 P=0.889
BLOOD GLUCOSE (mg/dL)
(C)
UPTAKES IN BRAIN (SUV)
R2=0.026 P=0.232
BLOOD TOTAL CHOLESTEROL (mg/dL)
(D)
UPTAKES IN BRAIN (SUV)
R2=0.206 P=0.0004
BLOOD TRIGLYCERIDE (mg/dL)
(E)
UPTAKES IN BRAIN (SUV)
R2=0.065 P=0.059
BLOOD INSULIN (ng/dL)

Fig.6
(A)
UPTAKES IN BROWN FAT CELLS (SUV)
R2=0.557 P<0.001
BUN/CRE
(B)
UPTAKES IN BROWN FAT CELLS (SUV)
R2=0.0007 P=0.897
BLOOD GLUCOSE (mg/dL)
(C)
UPTAKES IN BROWN FAT CELLS (SUV)
R2=0.023 P=0.470
BLOOD TOTAL CHOLESTEROL (mg/dL)
(D)
UPTAKES IN BROWN FAT CELLS (SUV)
R2=0.492 P<0.001
BLOOD TRIGLYCERIDE (mg/dL)
(E)
UPTAKES IN BROWN FAT CELLS (SUV)
R2=0.491 P<0.001
BLOOD INSULIN (ng/dL)

Fig.7
(A)
UPTAKES IN HEART (SUV)
R2=0.121 P=0.027
BUN/CRE
(B)
UPTAKES IN HEART (SUV)
R2=0.015 P=0.382
BLOOD GLUCOSE (mg/dL)
(C)
UPTAKES IN HEART (SUV)
R2=0.063 P=0.068
BLOOD TOTAL CHOLESTEROL (mg/dL)
(D)
UPTAKES IN HEART (SUV)
R2=0.065 P=0.065
BLOOD TRIGLYCERIDE (mg/dL)
(E)
UPTAKES IN HEART (SUV)
R2=0.068 P=0.059
BLOOD INSULIN (ng/dL)

Fig.8
(A)
UPTAKES IN PANCREAS (SUV)
R2=0.280 P<0.001
BUN/CRE
(B)
UPTAKES IN PANCREAS (SUV)
R2=0.016 P=0.347
BLOOD GLUCOSE (mg/dL)
(C)
UPTAKES IN PANCREAS (SUV)
R2=0.017 P=0.330
BLOOD TOTAL CHOLESTEROL (mg/dL)
(D)
UPTAKES IN PANCREAS (SUV)
R2=0.063 P=0.064
BLOOD TRIGLYCERIDE (mg/dL)
(E)
UPTAKES IN PANCREAS (SUV)
R2=0.068 P=0.053
BLOOD INSULIN (ng/dL)

Fig.9
(A)
UPTAKES IN LIVER (SUV)
0 2 4 6 8
R2=0.222 P=0.002
0 50 100 150 200
BUN/CRE
(B)
UPTAKES IN LIVER (SUV)
0 2 4 6 8
R2=0.034 P=0.134
0 200 400 600 800
BLOOD GLUCOSE (mg/dL)
(C)
UPTAKES IN LIVER (SUV)
0 2 4 6 8
R2=0.061 P=0.072
0 100 200 300 400 500
BLOOD TOTAL CHOLESTEROL (mg/dL)
(D)
UPTAKES IN LIVER (SUV)
0 2 4 6 8
R2=0.377 P<0.001
0 200 400 600 800 1000
BLOOD TRIGLYCERIDE (mg/dL)
(E)
UPTAKES IN LIVER (SUV)
0 2 4 6 8
R2=0.071 P=0.053
0 5 10 15 20
BLOOD INSULIN (ng/dL)

Fig.10
(A)
UPTAKES IN KIDNEY (SUV)
R2=0.117 P=0.003
BUN/CRE
(B)
UPTAKES IN KIDNEY (SUV)
R2<0.001 P=0.994
BLOOD GLUCOSE (mg/dL)
(C)
UPTAKES IN KIDNEY (SUV)
R2=0.058 P=0.080
BLOOD TOTAL CHOLESTEROL (mg/dL)
(D)
UPTAKES IN KIDNEY (SUV)
R2=0.067 P=0.060
BLOOD TRIGLYCERIDE (mg/dL)
(E)
UPTAKES IN KIDNEY (SUV)
R2=0.064 P=0.066
BLOOD INSULIN (ng/dL)

*Fig.11*

(A)
UPTAKES IN HEART (SUV)
25
20
15
10
5
0
40
60
80
100
120
BUN/CRE
R2=0.684 P<0.001

(B)
UPTAKES IN LIVER (SUV)
12
10
8
6
4
2
0
40
60
80
100
120
BUN/CRE
R2=0.601 P<0.001

(C)
UPTAKES IN KIDNEY (SUV)
12
10
8
6
4
2
0
40
60
80
100
120
BUN/CRE
R2=0.049 P=0.321

*Fig.12*

(A)

UPTAKES IN HEART
(SUV)
15
10
5
0
40 60 80 100 120 140
BUN/CRE
R2=0.698 P<0.001

(B)

UPTAKES IN LIVER
(SUV)
8
6
4
2
0
40 60 80 100 120 140
BUN/CRE
R2=0.632 P<0.001

(C)

UPTAKES IN KIDNEY
(SUV)
8
6
4
2
0
40 60 80 100 120 140
BUN/CRE
R2=0.033 P=0.416

## INTERNATIONAL SEARCH REPORT

International application No.
**PCT/JP2021/046814**

**A. CLASSIFICATION OF SUBJECT MATTER**

***G01N 33/50***(2006.01)i
FI: G01N33/50 Z

According to International Patent Classification (IPC) or to both national classification and IPC

**B. FIELDS SEARCHED**

Minimum documentation searched (classification system followed by classification symbols)

G01N33/50

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN); 医中誌WEB (Ichushi WEB)

**C. DOCUMENTS CONSIDERED TO BE RELEVANT**

| Category* | Citation of document, with indication, where appropriate, of the relevant passages | Relevant to claim No. |
|---|---|---|
| A | US 2020/0003762 A1 (BIOELECTRON TECHNOLOGY CORPORATION) 02 January 2020 (2020-01-02)<br>claims, paragraphs [0109]-[0110], table 5 | 1-3 |
| A | CHIU, P. Y. et al. Schisandrin B Enhances Renal Mitchondrial Antioxidant Status, Functional and Structural Integrity, and Protects against Gentamicin-Induced Nephrotoxicity in Rats. Bilogical and Pharmaceutical Bulletin. 2008, vol. 31, no. 4, pp. 602-605<br>in particular, "abstract", "results", fig. 1 | 1-3 |
| A | JP 2019-506374 A (SANOFI-AVENTIS DEUTSCHLAND GMBH) 07 March 2019 (2019-03-07)<br>paragraph [0059] | 1-3 |
| A | CN 111481510 A (NANKAI UNIVERSITY) 04 August 2020 (2020-08-04)<br>paragraphs [0032]-[0036] | 1-3 |
| A | JP 2017-206457 A (HAMAMATSU PHOTONICS KK) 24 November 2017 (2017-11-24)<br>paragraphs [0050]-[0054] | 1-3 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 January 2022** | **01 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2021/046814

C. DOCUMENTS CONSIDERED TO BE RELEVANT

| Category* | Citation of document, with indication, where appropriate, of the relevant passages | Relevant to claim No. |
|---|---|---|
| A | WO 2014/030709 A1 (HAMAMATSU PHOTONICS KK) 27 February 2014 (2014-02-27) paragraph [0123], fig. 10 | 1-3 |
| A | 鈴木透理　ほか, 腎病変を合併したミトコンドリア脳筋症(MELAS)の一例, 日本腎臓学会誌, 1996, vol. 38, no. 2, pp.109-114 in particular, "introduction", "cases", (SUZUKI, Tori et al. A case of mitochondrial encephalomyopathy (MELAS). The Japanese Journal of Nephrology.) | 1-3 |
| A | JP 2009-535061 A (THERAKOS, INC) 01 October 2009 (2009-10-01) claims, paragraph [0089] | 1-3 |

Form PCT/ISA/210 (second sheet) (January 2015)

**INTERNATIONAL SEARCH REPORT**
**Information on patent family members**

International application No.
**PCT/JP2021/046814**

| Patent document cited in search report | Publication date (day/month/year) | Patent family member(s) | Publication date (day/month/year) |
|---|---|---|---|
| US 2020/0003762 A1 | 02 January 2020 | (Family: none) | |
| JP 2019-506374 A | 07 March 2019 | US 2019/0002977 A1<br>paragraph [0134]<br>WO 2017/108941 A1<br>EP 3184106 A1 | |
| CN 111481510 A | 04 August 2020 | (Family: none) | |
| JP 2017-206457 A | 24 November 2017 | (Family: none) | |
| WO 2014/030709 A1 | 27 February 2014 | US 2015/0225368 A1<br>paragraphs [0194]-[0195], fig. 10<br>EP 2915809 A1<br>CN 104583192 A | |
| JP 2009-535061 A | 01 October 2009 | US 2007/0264663 A1<br>claims, paragraph [0084]<br>WO 2007/127493 A2<br>CN 101484586 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005115971 A

### Non-patent literature cited in the description

- *J. Nucl. Med.,* 2020, vol. 61, 96-103
- *Life Sciences,* 2005, vol. 76, 1825-1834
- *Clin. Kidney J.,* 2012, vol. 5, 187-191
- *Intensive Care Med.,* 2019, vol. 45, 1813-1815
- *Nephrology Reviews,* 2010, vol. 2, e11
- *J. Labeled Comp. Radiopharm.,* 2013, vol. 56 (11), 553-561